# EUROPÄISCHE PATENTSCHRIFT

(11) **EP 3 050 544 B1**
(45) Veröffentlichungstag und Bekanntmachung des Hinweises auf die Patenterteilung: **27.06.2018**
(21) Anmeldenummer: 16150814.8
(22) Anmeldetag: 13.03.2008
(51) Int. Cl.: A61F 9/008

(54) **VORRICHTUNG UND VERFAHREN ZUR MATERIALBEARBEITUNG MITTELS LASERSTRAHLUNG**
DEVICE AND METHOD FOR MATERIALS PROCESSING USING LASER RADIATION
DISPOSITIF ET PROCEDE DE TRAITEMENT DE MATERIAU A L'AIDE D'UN RAYON LASER

(30) Priorität: 11.04.2007 DE 102007017119
(43) Veröffentlichungstag der Anmeldung: 03.08.2016
(62) Teilanmeldung aus: 08716514.8
(73) Patentinhaber: Carl Zeiss Meditec AG, 07745 Jena (DE)
(72) Erfinder: BISCHOFF, Mark, 07749 Jena (DE); MÜHLHOFF, Dirk, 07751 Jena (DE); STOBRAWA, Gregor, 07743 Jena (DE)
(74) Vertreter: Patentanwälte Geyer, Fehners & Partner mbB

(56) Entgegenhaltungen:
- WO-A2-03/082146
- DE-A1-102005 039 833
- US-A1- 2004 243 111

## Beschreibung

Die Erfindung bezieht sich auf eine Vorrichtung zur Materialbearbeitung mittels Laserstrahlung, mit einer Laserstrahlungsquelle, die gepulste Laserstrahlung zur Wechselwirkung mit dem Material abgibt, einer die gepulste Bearbeitungs-Laserstrahlung in das Material auf ein Wechselwirkungszentrum fokussierenden Optik, einer die Lage des Wechselwirkungszentrums im Material verstellenden Scaneinrichtung, wobei jeder Bearbeitungs-Laserpuls in einer das ihm zugeordnete Wechselwirkungszentrum umgebenden Zone mit dem Material wechselwirkt, so dass in den Wechselwirkungszonen Material getrennt wird, und einer Steuereinrichtung, welche die Scaneinrichtung und die Laserstrahlquelle so ansteuert, dass im Material durch Aneinanderreihen von Wechselwirkungszonen eine Schnittfläche entsteht.

Die Erfindung bezieht sich ferner auf ein Verfahren zur nicht-chirurgischen Materialbearbeitung mittels Laserstrahlung, bei dem gepulste Bearbeitungs-Laserstrahlung erzeugt, zur Wechselwirkung ins Material auf Wechselwirkungszentren fokussiert wird, und die Lage der Wechselwirkungszentren im Material verstellt wird, wobei jeder Bearbeitungs-Laserpuls in einer Zone um das ihm zugeordnete Wechselwirkungszentrum mit dem Material wechselwirkt und in den Wechselwirkungszonen Material getrennt wird und eine Schnittfläche im Material durch Aneinanderreihen von Wechselwirkungszonen erzeugt wird.

Diese Vorrichtungen sowie entsprechende Verfahrenen zur Materialbearbeitung eignen sich besonders, um gekrümmte Schnittflächen innerhalb eines transparenten Materials auszubilden. Gekrümmte Schnittflächen innerhalb eines transparenten Materials werden beispielsweise bei laserchirurgischen Verfahren und dort insbesondere bei augenchirurgischen Eingriffen erzeugt. Dabei wird Behandlungs-Laserstrahlung in das Gewebe, d.h. unterhalb der Gewebeoberfläche auf ein Wechselwirkungszentrum fokussiert. In einer darum liegenden Wechselwirkungszone werden dadurch Materialschichten getrennt. Die Zone entspricht in der Regel dem Fokusspot. Üblicherweise wird die Laserpulsenergie so gewählt, dass in der Wechselwirkungszone ein optischer Durchbruch im Gewebe entsteht.

Im Gewebe laufen nach einen optischen Durchbruch zeitlich hintereinander mehrere Prozesse ab, die durch den Laserstrahlungspuls initiiert werden. Der optische Durchbruch erzeugt zuerst im Material eine Plasmablase. Diese Plasmablase wächst nach Entstehen durch sich ausdehnendes Gas. Anschließend wird das in der Plasmablase erzeugte Gas vom umliegenden Material aufgenommen und die Blase verschwindet wieder. Dieser Vorgang dauert allerdings sehr viel länger, als die Entstehung der Blase selbst. Wird ein Plasma an einer Materialgrenzfläche erzeugt, die durchaus auch innerhalb einer Materialstruktur liegen kann, so erfolgt ein Materialabtrag von der Grenzfläche. Man spricht dann von Photoablation. Bei einer Plasmablase, die vorher verbundene Materialschichten trennt, ist üblicherweise von Photodisruption die Rede. Der Einfachheit halber werden all solche Prozesse hier unter dem Begriff Wechselwirkung zusammengefasst, d.h. dieser Begriff schließt nicht nur den optischen Durchbruch sondern auch die anderweitigen materialtrennenden Wirkungen ein.

Für eine hohe Genauigkeit eines laserchirurgischen Verfahrens ist es unumgänglich, eine hohe Lokalisierung der Wirkung der Laserstrahlen zu gewährleisten und Kollateralschäden in benachbartem Gewebe möglichst zu vermeiden. Es ist deshalb im Stand der Technik üblich, die Laserstrahlung gepulst anzuwenden, so dass der zur Auslösung eines optischen Durchbruchs nötige Schwellwert für die Energiedichte nur in den einzelnen Pulsen überschritten wird. Die US-A-5984916 zeigt diesbezüglich deutlich, dass die räumliche Ausdehnung der Wechselwirkungszone nur dann wesentlich von der Pulsdauer abhängt, solange eine Pulslänge von 2 ps überschritten ist. Bei Werten von wenigen 100 fs ist die Wechselwirkungszonengröße nahezu pulsdauerunabhängig. Eine hohe Fokussierung des Laserstrahls in Kombination mit sehr kurzen Pulsen, d.h. unter 1 ps, erlaubt es damit, die Wechselwirkungszone punktgenau in einem Material einzusetzen.

Der Einsatz von solcher gepulster Laserstrahlung hat sich in der letzten Zeit besonders zur laserchirurgischen Fehlsichtigkeitskorrektur in der Ophthalmologie durchgesetzt. Fehlsichtigkeiten des Auges rühren oftmals daher, dass die Brechungseigenschaften von Hornhaut und Linse keine optimale Fokussierung auf der Netzhaut bewirken. Diese Art der Pulsung ist auch Gegenstand der hier geschilderten Erfindung.

Die erwähnte US-A-5984916 und auch die US-A-2004/0243111 beschreibt ein Verfahren zur Schnitterzeugung mittels geeigneter Erzeugung optischer Durchbrüche, so dass im Endeffekt die Brechungseigenschaften der Hornhaut gezielt beeinflusst werden. Eine Vielzahl von optischen Durchbrüchen wird so aneinandergesetzt, dass die Schnittfläche innerhalb der Hornhaut des Auges ein linsenförmiges Teilvolumen isoliert. Das vom übrigen Hornhautgewebe getrennte linsenförmige Teilvolumen wird dann über einen seitlich öffnenden Schnitt aus der Hornhaut herausgenommen. Die Gestalt des Teilvolumens ist so gewählt, dass nach Entnahme die Form und damit die Brechungseigenschaften der Hornhaut so geändert sind, dass die erwünschte Fehlsichtigkeitskorrektur bewirkt ist. Die dabei geforderte Schnittfläche ist gekrümmt und umschreibt das Teilvolumen, was eine dreidimensionale Verstellung des Fokus nötig macht. Es wird deshalb eine zweidimensionale Ablenkung der Laserstrahlung mit gleichzeitiger Fokusverstellung in einer dritten Raumrichtung kombiniert. Dies wird hier unter dem Begriff "scannen", "verstellen" oder "ablenken" subsumiert.

Die WO-A-2003/082146 beschreibt den Einsatz von Laserstrahlung zum Lösen einer Hornhautlamelle.

Beim Aufbau der Schnittfläche durch Aneinanderreihen optischer Durchbrüche im Material verläuft die Erzeugung eines optischen Durchbruches um ein Vielfaches schneller, als es dauert, bis ein davon erzeugtes Plasma wieder im Gewebe absorbiert wird. Aus der Veröffentlichung A. Heisterkamp et al., Der Ophthalmologe, 2001, 98:623-628, ist es bekannt, dass nach Erzeugen eines optischen Durchbruches in der Augenhornhaut am Fokuspunkt, an dem der optische Durchbruch erzeugt wurde, eine Plasmablase entsteht, die mit Nachbarblasen zu Makroblasen zusammenwachsen kann. Die Veröffentlichung führt aus, dass das Zusammenschließen noch anwachsender Plasmablasen die Schnittqualität mindert. Es wird deshalb dort ein gattungsgemäßes Verfahren vorgeschlagen, bei dem einzelne Plasmablasen nicht direkt nebeneinander erzeugt werden. Stattdessen wird in einer spiralförmigen Bahn zwischen aufeinanderfolgend erzeugten optischen Durchbrüchen eine Lücke gelassen, die in einem zweiten Durchlauf durch die Spirale mit optischen Durchbrüchen und daraus resultierenden Plasmablasen gefüllt wird. Damit soll ein Zusammenschluss benachbarter Plasmablasen während des Anwachsens verhindert und die Schnittqualität verbessert werden.

Um eine gute Schnittqualität zu erreichen, verwendet der Stand der Technik also bestimmte Abfolgen, in denen die optischen Durchbrüche erzeugt werden. So soll ein Zusammenschließen anwachsender Plasmablasen verhindert werden. Da natürlich ein Schnitt angestrebt ist, bei dem möglichst wenig Brücken das Material bzw. Gewebe verbinden, müssen letztlich die erzeugten Plasmablasen auf jeden Fall zu einer Schnittfläche zusammenwachsen. Ansonsten blieben Materialverbindungen und der Schnitt wäre unvollständig. Die DE 102005039833 A1 schlägt dazu vor, die Laserpulsenergie unter die Durchbruchsschwelle abzusenken und mehrere Laserpulse überdeckend und zeitlich direkt hintereinander ins Gewebe abzugeben.

Der Erfindung liegt deshalb die Aufgabe zugrunde, die Schnitte guter Qualität im Material zu erzeugen, ohne auf bestimmte Abfolgen der Laserpulseinbringung festgelegt zu sein.

Die Erfindung ist in den unabhängigen Ansprüchen definiert.

In einer ersten Variante einer Vorrichtung steuert die Steuereinrichtung die Laserstrahlquelle und die Scaneinrichtung so an, dass benachbarte Wechselwirkungszentren in einem örtlichen Abstand a ≤ 10 µm zueinander liegen. In der ersten Variante eines Verfahrens liegen benachbarte Wechselwirkungszentren in einem örtlichen Abstand a ≤ 10 µm.

In einer zweiten Variante einer Vorrichtung liegt die Fluence F der Pulse für jedes Wechselwirkungszentrum jeweils unter 5 J/cm². In der zweiten Variante eines Verfahren liegt für die Wechselwirkungszonen die Fluence F jeweils unter 5 J/cm².

In einer dritten Variante einer Vorrichtung steuert die Steuereinrichtung die Laserstrahlquelle und die Scaneinrichtung so an, dass die Schnittfläche zwei entlang der optischen Achse benachbarte Abschnitte aufweist und diese zumindest teilweise in einem zeitlichen Abstand t ≤ 5 s mit Laserpulsen beleuchtet. In der dritten Variante eines Verfahren weist die Schnittfläche zwei entlang der optischen Achse benachbarte Abschnitte auf und diese werden zumindest teilweise in einem zeitlichen Abstand t ≤ 5 s mit Laserpulsen beaufschlagt.

Die Varianten gehen von der Erkenntnis aus, dass sich Wechselwirkungszonen im Material gegenseitig beeinflussen. Die Wirkung eines Laserstrahlpulses hängt also davon ab, inwieweit in der Umgebung des Wechselwirkungszentrums bereits vorangegangene Lasereinwirkungen stattfanden. Daraus folgerten die Erfinder, dass die zur Erzeugung eines optischen Durchbruchs bzw. zur Bewirkung einer Materialtrennung erforderliche Pulsenergie vom Abstand zum nächstgelegenen Wechselwirkungszentrum abhängt. Die Varianten nutzen sämtlich diese Erkenntnis.

Die Minimierung des Abstandes von Wechselwirkungszentren, z. B. des Abstandes der Fokuslage benachbarter optischer Durchbrüche, gemäß Variante 1 ermöglicht eine Absenkung der Bearbeitungs-Pulsenergie. Der die Pulsenergie beschreibende Parameter ist die Fluence, also die Energie pro Fläche bzw. Energieflächendichte. Die Variante 1 mit einem Abstand unter 10 µm spricht also einen Aspekt der den Erfindern erstmals zuzuschreibenden Erkenntnis an.

Ein anderer Aspekt liegt darin, dass nun die Fluence der Bearbeitungs-Laserpulse deutlich gesenkt wird. Variante 2 widmet sich also dem gleichen Aspekt wie Variante 1, schreibt aber nun keine Obergrenze für den Abstand, sondern für die Fluence vor.

Die genannten Varianten stellen Rahmenbedingungen für die Erzeugung eines Schnittes durch Einbringung gepulster Laserstrahlung auf, wobei die Rahmenbedingungen die Auswirkungen des unmittelbar benachbarten eingebrachten Pulses berücksichtigen. Für die Pulslänge wird hier die Lehre der US-A-5984916 angewendet, also Pulse unter 1 ps, vorzugsweise wenige 100 fs, z. B. 300 - 500 fs. Soweit Varianten eine Obergrenze des Abstandes definieren, ist der Abstand zum örtlich nächst gelegenen Wechselwirkungszentrum gemeint. Da eine Schnittfläche in der Regel durch eine Vielzahl von aneinandergereihten Wechselwirkungszentren erzeugt wird, kann unter dem Abstand zur Vereinfachung auch der Mittelwert des Abstandes von Laserfoki für die einzelnen Laserpulse im Material verstanden werden. Wenn das entlang einer Schnittfläche im Wesentlichen zweidimensionale Gitter aus Wechselwirkungszentren nicht symmetrisch ist, kann mit dem Abstand auch der charakteristische mittlere Abstand gemeint sein. Im Stand der Technik ist es bekannt, eine gepulste Laserstrahlungsquelle zu verwenden und einen Teil der davon abgegebenen Laserstrahlungsquelle so zu verändern, dass sie keinen Bearbeitungseffekt im Material auslösen. Nur ein Teil der Laserstrahlungspulse dient dann zur Bearbeitung. Soweit für die hier vorliegende Beschreibung des Begriffs "Laserstrahlungspuls", "Laserpuls" oder "Puls" verwendet wird, ist immer ein Bearbeitungs-Laserpuls gemeint, also ein Laserstrahlungspuls der zur Wechselwirkung mit dem Material vorgesehen bzw. ausgebildet bzw. geeignet ist.

Der apparative Aufwand sinkt durch Varianten, weil die Pulsspitzenleistung abnimmt. Durch den reduzierten Abstand der Wechselwirkungszentren steigt die Pulsfolgefrequenz, wenn die Bearbeitungsdauer konstant gehalten wird. Weiter werden im Falle optischer Durchbrüche kleinere Plasmablasen erzeugt, wodurch der Schnitt feiner wird. Im Stand der Technik wurde hingegen immer mit vergleichsweise größeren Abständen der Wechselwirkungszentren gearbeitet und die Fluence der Pulse entsprechend hoch gewählt, um gesichert optische Durchbrüche und entsprechend an die Abstände angepasste, große Plasmablasen zu erhalten.

Eine niedrigere Fluence reduziert zugleich auch die Personengefährdung bei der Materialbearbeitung. Bei augenchirurgischen Verfahren ist dies von wesentlicher Bedeutung. Als besonderer Vorteil stellt sich dabei heraus, dass nunmehr mit Lasern der Gefährdungsklasse 1M gearbeitet werden kann, wohingegen im Stand der Technik die Laserklasse 3 nötig war. Diese Klasse verlangte, dass Bedienpersonal, beispielsweise ein Arzt oder eine Krankenschwester, Schutzbrillen tragen, was von Patienten naturgemäß als beunruhigend empfunden wird. Solche Schutzmaßnahmen sind mit den erfindungsgemäß nun möglichen Laser der Klasse 1M nicht mehr nötig.

Es ist deshalb als Weiterbildung ebenfalls vorgesehen eine Vorrichtung zur Materialbearbeitung mittels Laserstrahlung, mit einer abgebenden Laserstrahlquelle, die gepulste Laserstrahlung zur Wechselwirkung mit dem Material abgibt, einer die gepulste Laserstrahlung in das Material auf ein Wechselwirkungszentrum fokussierenden Optik, einer die Lage des Wechselwirkungszentrums im Material verstellenden Scaneinrichtung, wobei jeder Bearbeitungs-Laserpuls in einer das ihm zugeordnete Wechselwirkungszentrum umgebenden Zone mit dem Material wechselwirkt, so dass in den Wechselwirkungszonen Material getrennt wird, mit und einer Steuereinrichtung, welche die Scaneinrichtung und die Laserstrahlquelle so ansteuert, dass im Material durch Aneinanderreihen von Wechselwirkungszonen eine Schnittfläche entsteht, wobei ein Laser einer Gefährdungsklasse unter 3, vorzugsweise ein Laser der Gefährdungsklasse 1M, zum Einsatz kommt. Die Angabe der Gefährdungsklasse bezieht sich auf die internationale Norm IEC 60825-1 in der am 13. Oktober 2005 geltenden Fassung. Analog ist (als Weiterbildung) vorgesehen eine Vorrichtung zur Materialbearbeitung mittels Laserstrahlung, mit einer Laserstrahlquelle, die gepulste Laserstrahlung zur Wechselwirkung mit dem Material abgibt, einer die gepulste Laserstrahlung entlang einer optischen Achse in das Material auf ein Wechselwirkungszentrum fokussierenden Optik, einer die Lage des Wechselwirkungszentrums im Material verstellenden Scaneinrichtung wobei jeder Laserpuls in einer das zugeordnete Wechselwirkungszentren umgebenden Zone mit dem Material wechselwirkt und in den Wechselwirkungszonen Material getrennt wird, und mit einer Steuereinrichtung, welche die Scaneinrichtung und die Laserstrahlquelle so ansteuert, dass im Material durch Aneinanderreihen von Wechselwirkungszonen eine Schnittfläche entsteht, wobei ein Laser einer Gefährdungsklasse unter 3, vorzugsweise ein Laser der Gefährdungsklasse 1M, verwendet wird. Dies bietet sich auch als Weiterbildung für jede der genannten Vorrichtungen bzw. für jedes der genannten Verfahren an. Soweit nicht explizit ausgeführt, gilt dies nachfolgend für jede geschilderte vorteilhafte Ausgestaltung, Weiterbildung oder Ausführung.

Von den Erfindern durchgeführte Untersuchungen zeigten, dass ein optischer Durchbruch erst oberhalb eines bestimmten Schwellwertes M einsetzt, der vom Abstand a benachbarter Wechselwirkungszentren gemäß der Gleichung M = 3,3 J/cm² - (2,4 J/cm²) / (1 + (a/r)²) abhängt. Erst bei einer Puls-Fluence über dem Schwellwert M ist ein optischer Durchbruch durch jeden einzelnen Laserpuls gesichert. Der in der Gleichung auftretende Parameter r stellt dabei eine experimentell erkannte mittlere Reichweite der Beeinflussung benachbarter Wechselwirkungszonen dar. Applikationsabhängig können hier Schwankungen vorliegen, so dass eine Variation des Wertes zwischen 3 und 10 µm möglich ist, vorzugsweise gilt r = 5 µm. Die für Variante 2 erwähnte Obergrenze der Puls-Fluence wird in einer Weiterbildung auch auf die genannte Abhängigkeit des Schwellwertes vom Abstand benachbarter Wechselwirkungszentren bezogen werden. Es ist deshalb eine Weiterbildung bevorzugt, bei der die Fluence um eine Überschussenergie von maximal 3 J/cm² über dem Schwellwert M liegt. Der dadurch definierte Bereich liefert eine besonders gute Schnittqualität, gleichzeitig ist die Einleitung eines optischen Durchbruches sichergestellt. Würde man die Überschussenergie weiter vergrößern, würden unnötig große Plasmablasen erzeugt und die Schnittqualität verschlechterte sich.

Es muss zur Schnitterzeugung nun aber nicht mehr zwingend mit optischen Durchbrüchen gearbeitet werden. Auch mit Energie der gepulsten Laserstrahlung, die unter einem Schwellwert zur Einleitung eines optischen Durchbruches liegt, kaum nach Erkenntnis der Erfinder eine Materialtrennung und damit eine Schnittflächenbildung erfolgen, wenn sich Wechselwirkungszonen überlappen. Es ist deshalb eine Weiterbildung vorgesehen, bei der der örtliche Abstand a der Wechselwirkungszentren zweier aufeinanderfolgender Pulse kleiner ist als die Fokusgröße d, so dass aufeinanderfolgend mit Laserstrahlung beaufschlagte Volumina des Materials, also Wechselwirkungszonen, sich gegenseitig überlappen. Durch diese Ausgestaltung erfolgt Materialtrennung ohne Plasmablasenbildung, was zu einem besonders glatten Schnitt führt.

Vorteilhafterweise kann dann die Fluence des Laserpulses auch unter den bereits erläuterten Schwellwert abgesenkt werden, da durch Überlagerung von Wechselwirkungszonen insgesamt dennoch ein gewebetrennender Effekt erreicht wird. Der einzelne Laserpuls erzeugt dann nicht mehr sicher einen optischen Durchbruch; erst die Überlagerung von Wechselwirkungszonen bewirkt die Gewebetrennung. Dies erlaubt Puls-Energien, die um Größenordnungen unter den bislang üblichen liegen, zugleich wird die Schnittqualität nochmals gesteigert, da sich zeitlich aufeinanderfolgend erzeugte Wechselwirkungszonen überdecken. Der Abstand der Wechselwirkungszentren reicht also von Null bis zum Durchmesser des Fokus, welcher beispielsweise zwischen 1 und 5 µm liegt, wenn man den 1/e²-Durchmesser (e = Eulersche Zahl) betrachtet.

Diese Schnitterzeugung bewirkt einen sehr feinen Schnitt, da aufgrund des reduzierten Abstandes bzw. der reduzierten Pulsenergie mit entsprechend kleinen oder sogar ganz ohne Plasmablasen gearbeitet wird bzw. gearbeitet werden kann. Eine feine Schnittfläche kann aber auch nachteilig sein, beispielsweise wenn ein Bediener die Schnittfläche zumindest teilweise optisch erkennen muss. Dies ist beispielsweise bei der Laserchirurgie nach dem fs-LASIK-Verfahren der Fall. Das dort durch die Einwirkung der Laserstrahlung isolierte Teilvolumen, das durch einen seitlichen Schnitt aus dem Gewebe entnommen werden soll, wird üblicherweise durch einen Operateur zuerst mittels eines Spatels von etwaigen Restbrücken zum umgebenden Material befreit. Dazu schiebt der Operateur den Spatel in die durch den seitlich öffnenden Schnitt erzeugte Tasche und fährt das Teilvolumen mit dem Spatel ab. Bei einer sehr feinen, also glatten Schnittfläche kann es vorkommen, dass der Operateur den Verlauf der Schnittfläche im Material nicht mehr von außen sehen kann. Er weiß deshalb nicht, wo der Rand des Teilvolumens liegt, und kann den Spatel nicht sicher führen. Zur Lösung dieser Problematik ist ein Verfahren der eingangs genannten Art vorgesehen, bei dem die Schnittfläche in mindestens zwei Teilflächen aufgeteilt wird und eine Teilfläche mit Betriebsparametern erzeugt wird, die eine gröbere also rauhere Schnittfläche erzeugen. Bei einer gattungsgemäßen Vorrichtung führt die Steuereinrichtung die entsprechende Ansteuerung von Laserquelle und Scaneinrichtung aus. Vorzugsweise wird man diese gröbere Schnittfläche an den Rand legen, der dadurch für den Benutzer einfach erkennbar ist und für die Qualität der Schnittfläche, z. B. bei der Augenchirurgie, keine Rolle spielt. Die beiden Teilflächen unterscheiden sich also hinsichtlich mindestens eines die Feinheit der Schnittfläche beeinflussenden Parameters. Ein möglicher Parameter ist beispielsweise die Fluence der verwendeten Laserpulse oder der örtliche Abstand der Wechselwirkungszentren. Ein anderer die Art der nachstehend noch erläuterten Perforation.

Kombiniert man diesen Ansatz, der prinzipiell auf verschiedene Art und Weisen ausgeführt werden kann und nicht auf die hier geschilderten Varianten beschränkt ist, mit einer der genannten Erfindungsvarianten, ist es zweckmäßig, dass die Steuereinrichtung die Laserstrahlquelle und die Scaneinrichtung so ansteuert, dass die Schnittfläche aus mindestens einer ersten und einer zweiten Teilschnittfläche aufgebaut ist, wobei die erste Teilschnittfläche mit einer Ansteuerung der Laserstrahlquelle und der Scaneinrichtung nach einem der vorgenannten Konzepte und die zweite Teilschnittfläche mit einer Ansteuerung der Laserstrahlquelle erfolgt, die eine Puls-Fluence über 3 J/cm² vorzugsweise über 5 J/cm² bewirkt. Dabei kann natürlich auch a > 10 µm eingestellt werden, da die Plasmablasen dann groß sind. Letztere Teilfläche weist dann automatisch die erwünschte gröbere Struktur auf und erleichtert dem Bediener bzw. Operateur das Erkennen der Schnittfläche. Das analoge Verfahren sieht entsprechend vor, dass die zweite Teilschnittfläche mit einem der erfindungsgemäßen Verfahren mit einer Puls-Fluence über 3 J/cm², vorzugsweise über 5 J/cm², erzeugt wird.

Zweckmäßigerweise wird man die gröbere Teilfläche so wählen, dass sie die feinere Teilfläche umgibt, so dass der Operateur den Rand der Schnittfläche gut erkennen kann und keine Nachteile für die optische Abbildung (im Falle der Augenchirurgie) am behandelten Auge entstehen.

Die der Erfindung zugrunde liegende Erkenntnis zeigt weiter, dass mit einem abnehmenden Abstand der Wechselwirkungszentren der Schwellwert, der zur gesicherten Erreichung eines optischen Durchbruchs nötig ist, abfällt.

Die von den Erfindern durchgeführte Analyse zeigte weiter, dass die Gestalt der erzeugten Plasmablasen, welche als Ergebnis der Wechselwirkung der Laserpulse mit dem Material bzw. Gewebe entstehen, einer zeitlichen Änderung unterliegen kann, wie es auch die Veröffentlichung von Heisterkamp et al. andeutete. Während diese Veröffentlichung sich aber darauf konzentriert, zu verhindern, dass ein Wechselwirkungszentrum nahe einer gerade anwachsenden Plasmablase liegt, hebt Variante 3 nun darauf ab, dass sich die Deformation einer Makroblase nicht auf die Schnittqualität auswirkt. Würde in ein deformiertes Material oder Gewebe an eine bestimmte Position ein weiterer optischer Durchbruch gesetzt, so verschöbe sich die Lage des Wechselwirkungszentrums im Material oder Gewebe, sobald die Deformation durch Relaxation zurückgeht. Es ist deshalb in der dritten Variante vorgesehen, die Zeit zwischen der Applikation der Laserenergie in zwei sich potentiell beeinflussenden Gebieten des Materials bzw. Gewebes so gering zu halten, dass sie kleiner ist, als eine charakteristische Zeit für die Bildung von Makroblasen. Diese liegt bei etwa 5 s. Dieses Vorgehen ist natürlich nur dann erforderlich, wenn zwei entlang der optischen Achse benachbarte Abschnitte der Schnittfläche vorhanden sind, da nur dann eine Deformation, die durch Erzeugung eines Schnittflächen-Abschnittes erzeugt wurde, sich auf die Ausbildung des anderen, entlang der optischen Achse benachbarten Schnittflächen-Abschnittes auswirken kann.

Besonders bedeutsam ist dieses Vorgehen bei der Erzeugung eines Teilvolumens beim fs-LASIK-Verfahren. Dieses auch als Lentikel bezeichnete Teilvolumen wird durch einen posterioren und einen anterioren Abschnitt der Schnittfläche erzeugt, so dass die Schnittfläche insgesamt das Lentikel umschreibt. Posterioren und anterioren Abschnitt zusammen innerhalb der charakteristischen Zeit zur Bildung der Makroblasen zu erzeugen, kann jedoch relativ hohe Anforderungen an die Ablenkgeschwindigkeit der Scaneinrichtung mit sich bringen oder erzwingt bestimmte Scanbahnen. Die kann vorzugsweise vermieden werden, wenn man den posterioren und den anterioren Abschnitt in Teilflächen aufteilt und die Bearbeitungsabfolge dieser Teilflächen geschickt wählt.

In einer Ausgestaltung werden die beiden Flächen in ringförmige Teilflächen unterteilt. Da bei einem Lentikel die zentrale Teilfläche die optische Qualität sehr viel stärker beeinflusst die Randgebiete, wird zuerst der Schnitt entsprechend der zentralen Teilfläche des posterioren Abschnittes und dann der des anterioren Abschnittes erzeugt, so dass die Teilflächen zeitlich unmittelbar benachbart gebildet werden. Dann wird die ringförmige Teilfläche des posterioren Abschnittes und anschließend des anterioren Abschnittes geschnitten. Dieses Prinzip kann auch mit beliebig vielen Teilflächen ausgeführt werden. Die praktischen Grenzen ergeben sich dadurch, dass zum Wechsel zwischen anteriorem und posteriorem Abschnitt immer der Laserfokus entlang der optischen Achse verändert werden muss, was aus technischen Gründen die meiste Zeit bei der Ablenkung in Anspruch nimmt.

Bei diesem Vorgehen ist es wichtig zu beachten, dass der Durchmesser einer jeden ring- bzw. kreisförmigen posterioren Teilfläche etwas größer sein muss, als der Durchmesser der anschließend erzeugten jeweiligen anterioren Teilfläche. Das stellt sicher, dass der als nächstes zu erzeugende, posteriore Teilschnitt nicht nur anterior liegende, als Streuzentren wirkende Disruptionsblasen unmöglich gemacht wird. Das Mindestmaß, um welches der posteriore Teilschnitt größer sein muss, als der zugeordnete anteriore Teilschnitt, ergibt sich aus der numerischen Apertur der fokussierenden Optik.

Eine weitere Möglichkeit, den zeitlichen Abstand unter die charakteristische Zeit zu drücken, besteht darin, den posterioren Abschnitt mit einer von außen nach innen verlaufenden Spirale der Wechselwirkungszentren zu erzeugen, und den anterioren Abschnitt mit einer von innen nach außen verlaufenden Spirale. Damit ist sichergestellt, dass entlang der optischen Achse benachbarte Abschnitte zumindest im zentralen Bereich innerhalb des zeitlichen Abstandes von 5 s gebildet werden. Dieses Verfahren lässt sich natürlich auf die bereits erwähnten Teilflächenaufteilungen anwenden.

Es ist deshalb bevorzugt, dass die Steuereinrichtung die Laserstrahlquelle sowie die Scaneinrichtung so ansteuert, dass die der optischen Achse benachbarten Abschnitte zumindest teilweise unmittelbar zeitlich aufeinanderfolgend durch das Aneinanderreihen der Wechselwirkungszentren beleuchtet.

Es kann bewusst von einer vollständigen Trennung zweier Materialbereiche Abstand genommen werden, indem bei der Materialbearbeitung in der Schnittfläche mehr oder weniger gleichmäßig verteilte Materialbrücken stehen gelassen werden. Diese Materialbrücken sorgen dafür, dass das Material an der Schnittfläche nur perforiert ist und noch über die Materialbrücken miteinander zusammenhängt. Das Perforationsmuster definiert die Schnittfläche. Größe und Zahl der Materialbrücken kann applikationsabhängig gewählt werden. Die Einbringung der Laserpulse wird so gesteuert bzw. erfolgt so, dass die Wechselwirkungszonen um die optischen Durchbrüche herum nicht vollständig zusammenwachsen. Die Wechselwirkung eines einzelnen Laserimpulses mit dem Material definiert eine räumliche Wechselwirkungszone in der Größenordnung des Laserfokus, in der es zu einer lokalen Trennung des Materials kommt. Durch lückenhafte Aneinanderreihung solcher Wechselwirkungszonen wird somit nun eine Perforation ausgebildet, die durch die Erhaltung von Materialbrücken zwischen benachbarten Wechselwirkungszonen gekennzeichnet ist. Die Perforation geht in eine Trennung über, wenn diese Materialbrücken alle verschwinden, was durch den räumlichen Überlapp benachbarter Wechselwirkungszonen erfolgen kann. Eine Perforation, bei der weiterhin Materialbrücken bestehen bleiben, hat den Vorteil, dass der Arzt die eigentliche Trennung manuell durchführt, der Trennvorgang also seiner bewussten Entscheidung überlassen bleibt. Er hat auf diese Weise die Möglichkeit, eine Perforation nicht zu trennen, die aus welchem Grund auch immer seiner beabsichtigten Therapie nicht entspricht.

Das bewusste Stehenlassen von Materialbrücken durch Perforation des Gewebes in einer Schnittfläche ohne vollständiges Ausbilden der Schnittfläche hat darüber hinaus den Vorteil, dass im Falle einer nicht wunschgemäßen Lage der derart vorbereiteten Schnittfläche eine Gewebstrennung noch nicht abgeschlossen ist, also keine unerwünschte endgültige Trennung von Materialbereichen, insbesondere Gewebeschichten, vorliegt.

Weiter zeigte sich überraschenderweise, dass die Ausbildung einer lediglich perforierten Schnittfläche und nachfolgende Trennung der Materialbrücken auf manuelle Art und Weise insgesamt zu einer glatteren Schnittfläche führt, gerade weil ein Zusammenwachsen von Wechselwirkungszonen nicht auftritt. Man kann somit besonders bevorzugt die vorstehend geschilderten Varianten der Laserpulseinbringung einsetzen, wobei die Laserpulszentren nun so liegen, dass nicht alle Wechselwirkungszonen zusammenwachsen, sondern zumindest zwischen einigen Wechselwirkungszonen Materialbrücken stehen bleiben. Die dafür mögliche Pulsenergieabsenkung hat den Vorteil, dass die Wechselwirkungszonen und insbesondere die Plasmablasen beim Einsatz im Gewebe, z.B. der Augenhornhaut, besonders klein sind, so dass sich nach dem Durchtrennen der Materialbrücken eine glattere Schnittfläche ergibt, als wenn man die Schnittfläche ohne Materialbrücken ausbildete. Die optische Qualität einer durch Perforation und nachträgliche manuelle Trennung ausgebildeten Schnittfläche ist also besser, als bei vollständiger Trennung der Gewebsschichten oder Materialbereiche durch eng aneinander liegende oder stark überlappende Wechselwirkungszonen. Diese Erkenntnis ist durchaus überraschend, da man im Stand der Technik bislang immer davon ausging, dass eine möglichst materialbrückenfreie Schnittflächenausbildung erforderlich sei und beim Stehenbleiben von Materialbrücken die optische Qualität gemindert wäre. Die neue erfinderische Erkenntnis kann besonders zweckmäßig dahingehend eingesetzt werden, dass in Bereichen, in denen eine hohe Qualität der Schnittfläche gefordert ist, mehr oder stabilere Materialbrücken stehen bleiben, als in anderen Bereichen. Die Zahl und/oder Dicke der Materialbrücken kann also bereichsabhängig variiert werden. Hierbei kann besonders zweckmäßig die eingangs sowie nachfolgend noch erwähnte Steuerung mit Datensätzen, welche unterschiedliche Betriebsparameter für einzelne Bereiche des zu bearbeitenden Materials/Gewebes vorgeben, eingesetzt werden.

Im Falle der Augenchirurgie ist es bekannt, zur refraktiven Korrektur ein linsenförmiges Volumen, auch als Lentikel bezeichnet, in der Hornhaut durch Ausbildung einer Schnittfläche zu isolieren und aus der Hornhaut zu entnehmen. Gängige Praxis ist es dabei zuerst die hornhautscheitelferner liegende Fläche des Lentikels zu schneiden und danach die hornhautscheitelnähere. Erster Schnitt wird auch als Lentikel-Schnitt, der zweite als Flap-Schnitt bezeichnet, wobei diese Bezeichnung sich an die herkömmliche, mikrokeratomgestützte Chirurgie anlehnt. Der Chirurg klappt dann die durch den Flap-Schnitt isolierte Hornhautlamelle ab und entnimmt das Lentikel. Bildet man die Schnittfläche, wie es im Rahmen dieser Erfindung auch vorgesehen sein kann, mit Materialbrücken, d.h. durch ein die Schnittfläche definierendes Perforationsmuster aus, ist es zweckmäßig, die Materialbrücken auf der Lentikelschnittseite stabiler und/oder zahlreicher auszubilden, als auf der Oberseite des Lentikels, d.h. am Flap-Schnitt. Dann kann der Chirurg die durch den Flap isolierte Lamelle zur Seite klappen und es ist nicht zu befürchten, dass das Lentikel an der Lamelle haftet. Es ist deshalb zweckmäßig, bei der refraktiven Augenchirurgie, eine Isolierung eines Lentikels durch mittels Perforation ausgebildeten Schnittflächen zu bewirken, wobei die Materialbrücken an der posterioren und der anterioren Fläche des Lentikels unterschiedlich so gestaltet werden, dass das Lentikel stärker an seiner posterioren Fläche haftet, als an der anterioren.

Die Ausbildung der Schnittfläche mittels Perforation kann prinzipiell mit jeder tauglichen Schnittflächenausbildung durch Einbringung gepulster Laserstrahlung erzeugt werden. Die an anderer Stelle hier beschriebenen Varianten und Gestaltungen der Laserpulseinbringung sind natürlich besonders vorteilhaft einsetzbar, jedoch nicht Voraussetzung.

Analoges gilt für die Ausgestaltung des erfindungsgemäßen Verfahrens.

Die Erfindung wird nachfolgend unter Bezugnahme auf die Zeichnungen beispielhalber noch näher erläutert. In den Zeichnungen zeigt:
- Fig. 1: ein laserchirurgisches Instrument zur Augenbehandlung,
- Fig. 2: eine Schemazeichnung der Wirkung der Laserstrahlung auf die Augenhornhaut beim Instrument der Fig. 1,
- Fig. 3: eine Schemadarstellung zur Veranschaulichung der Erzeugung und Isolierung eines Teilvolumens mit dem Instrument der Fig. 1,
- Fig. 4: eine Ablenkvorrichtung des Instruments der Fig. 1,
- Fig. 5: ein Blockdiagramm des Aufbaus des Instruments der Fig. 1,
- Fig. 6: einen Zusammenhang zwischen Abstand der Zentren der optischen Durchbrüche, die vom Instrument der Fig. 1 erzeugt werden, und der Pulsenergie, wobei mögliche Betriebsbereiche für das Instrument der Fig. 1 eingezeichnet sind,
- Fig. 7: eine Darstellung ähnlich der Fig. 6,
- Fig. 8: eine schematische Draufsicht auf die Augenhornhaut zur Verdeutlichung der Lage der erzeugten Plasmablasen bzw. der dadurch bewirkten Schnittfläche,
- Fig. 9: eine Schnittdarstellung durch die Darstellung der Fig. 8 gemäß der Linie A1-A1,
- Fig. 10: eine Schemadarstellung zur Anordnung mehrerer Wechselwirkungszonen bei der Erzeugung der Schnittfläche mit einem Instrument gemäß Fig. 1,
- Fig. 11 und 12: Darstellungen ähnlich der Fig. 10 für abgewandelte Betriebsmodi und
- Fig. 13a, b und 14: eine Drauf- und Schnittansicht einer Schnittfläche, die mit einem Instrument gemäß Fig. 1 ausgebildet wurde.

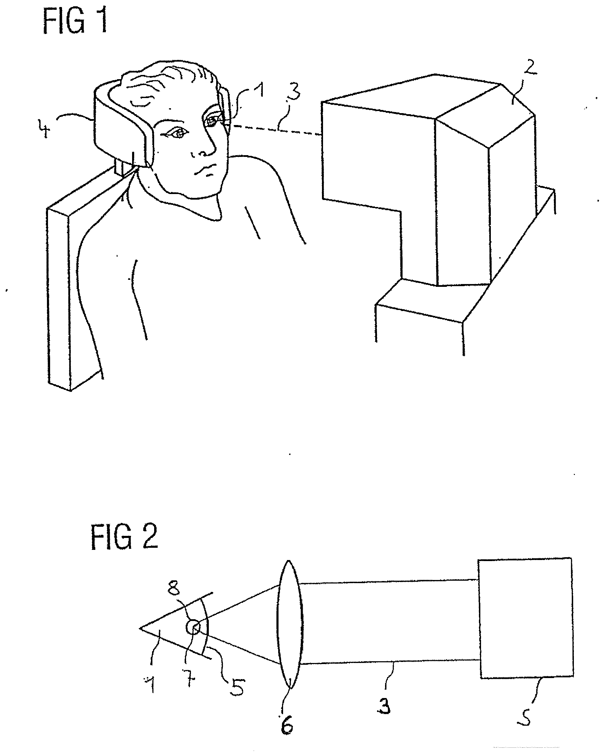

In Fig. 1 ist ein laserchirurgisches Instrument zur Behandlung eines Auges 1 eines Patienten gezeigt, wobei das laserchirurgische Instrument 2 zur Ausführung einer refraktiven Korrektur dient. Das Instrument 2 gibt dazu einen Behandlungs-Laserstrahl 3 auf das Auge des Patienten 1 ab, dessen Kopf in einem Kopfhalter 4 fixiert ist. Das laserchirurgische Instrument 2 ist in der Lage, einen gepulsten Laserstrahl 3 zu erzeugen, so dass das in US-A-5984916 beschriebene Verfahren ausgeführt werden kann. Der Behandlungs-Laserstrahl 3 besteht z. B. aus fs-Laserpulsen mit einer Pulswiederholfrequenz zwischen 10 und 500 kHz. Die Baugruppen des Instrumentes 2 werden im Ausführungsbeispiel von einer integrierten Steuereinheit gesteuert.

Das laserchirurgische Instrument 2 weist, wie in Fig. 2 schematisch dargestellt ist, eine Strahlquelle S auf, deren Strahlung in die Hornhaut 5 des Auges 1 fokussiert wird. Mittels des laserchirurgischen Instrumentes 2 wird eine Fehlsichtigkeit des Auges 1 des Patienten dadurch behoben, dass aus der Hornhaut 5 Material so entfernt wird, dass sich die Brechungseigenschaften der Hornhaut um ein gewünschtes Maß ändern. Das Material wird dabei dem Stroma der Hornhaut entnommen, das unterhalb von Epithel und Bowmanscher Membran und oberhalb von Decemetscher Membran und Endothel liegt.

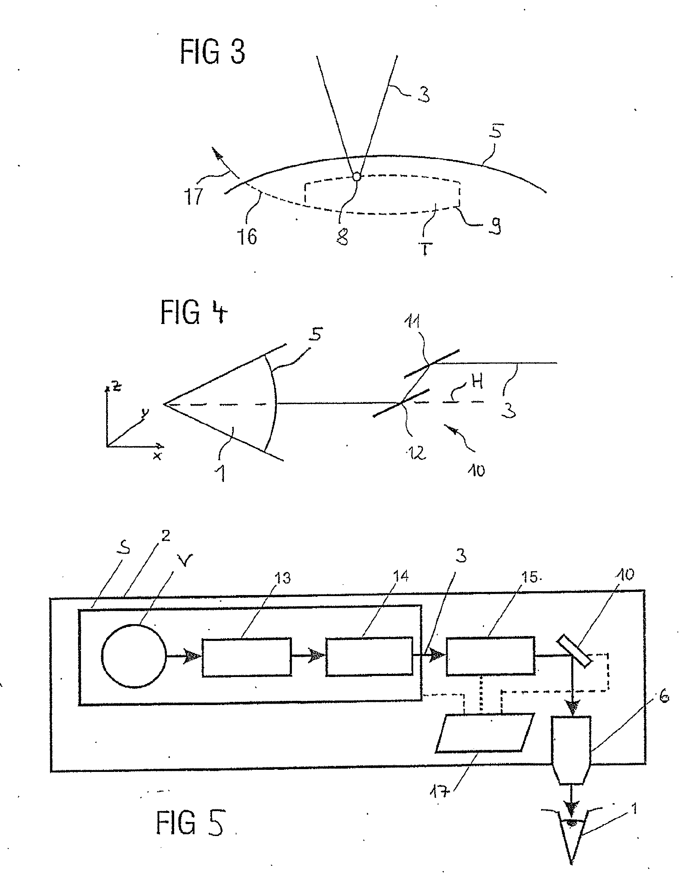

Die Materialentfernung erfolgt, indem durch Fokussierung des hochenergetischen gepulsten Laserstrahls 3 mittels eines verstellbaren Teleskopes 6 in einem in der Hornhaut 5 liegenden Fokus 7 in der Hornhaut Gewebeschichten getrennt werden. Jeder Puls der gepulsten Laserstrahlung 3 erzeugt dabei einen optischen Durchbruch im Gewebe, welcher wiederum eine Plasmablase 8 initiiert. Dadurch umfasst die Gewebeschichttrennung ein größeres Gebiet, als der Fokus 7 der Laserstrahlung 3, obwohl die Bedingungen zur Erzielung des Durchbruches nur im Fokus 7 erreicht werden. Durch geeignete Ablenkung des Laserstrahls 3 werden nun während der Behandlung viele Plasmablasen 8 erzeugt. Dies ist schematisch in Fig. 3 dargestellt. Die Plasmablasen bilden dann eine Schnittfläche 9, die ein Teilvolumen T des Stromas, nämlich das zu entfernende Material der Hornhaut 5 umschreiben. Durch Aneinanderreihung der Plasmablasen 8 in Folge stetiger Verschiebung des Fokus 7 des gepulsten fokussierten Laserstrahls 3 wird die Schnittfläche 9 gebildet.

Das laserchirurgische Instrument 2 wirkt durch die Laserstrahlung 3 wie ein chirurgisches Messer, das, ohne die Oberfläche der Hornhaut 5 zu verletzen, direkt Materialschichten im Inneren der Hornhaut 5 trennt. Führt man einen Schnitt 16 durch weitere Erzeugung von Plasmablasen 8 bis an die Oberfläche der Hornhaut, kann ein durch die Schnittfläche 9 isoliertes Material der Hornhaut 5 seitlich in Richtung des Pfeiles 17 herausgezogen und somit entfernt werden.

Die Fokusverschiebung erfolgt dabei zum einen in einer Ausführungsform mittels der in Fig. 4 schematisch dargestellten Ablenkeinheit 10, die den auf einer optischen Achse H auf das Auge 1 einfallenden Laserstrahl 3 um zwei senkrecht zueinander liegenden Achsen ablenkt. Die Ablenkeinheit 10 verwendet dafür einen Zeilenspiegel 11 sowie einen Bildspiegel 12, was zu zwei hintereinander liegenden räumlichen Ablenkachsen führt. Der Kreuzungspunkt der optischen Achse H mit der Ablenkachse ist dann der jeweilige Ablenkpunkt. Zur Fokusverschiebung wird zum anderen das Teleskop 6 geeignet verstellt. Dadurch kann der Fokus 7 in dem in Fig. 4 schematisch dargestelltem x/y/z-Koordinatensystem entlang dreier orthogonaler Achsen verstellt werden. Die Ablenkeinheit 10 verstellt den Fokus in der x/y-Ebene, wobei der Zeilenspiegel den Fokus in der x-Richtung und der Bildspiegel in der y-Richtung zu verstellen erlaubt. Das Teleskop 6 wirkt dagegen auf die z-Koordinate des Fokus 7. Somit ist insgesamt eine dreidimensionale Ablenkung des Fokus 7 erreicht.

Aufgrund der Cornea-Krümmung, die zwischen 7 und 10 mm beträgt, muss das Teilvolumen T auch entsprechend gekrümmt sein. Die Cornea-Krümmung erfordert somit eine der Schnittebene. Diese wird durch geeignete Ansteuerung der Ablenkeinheit 10 und des Teleskopes 6 bewirkt.

Die Fig. 5 zeigt ein vereinfachtes Blockschaltbild des laserchirurgischen Instrumentes 2 für die refraktiven Chirurgie am menschlichen Auge 1. Dargestellt sind nur die wichtigsten Baugruppen: ein als Strahlquelle S dienender fs-Laser, welcher aus einem fs-Oszillator V, sowie einer oder mehreren Verstärkerstufen 13 besteht und dem hier noch ein Kompressor bzw. Pre-Kompressor 14 nachgeordnet ist; ein Laserpulsmodulator 15, der mit der Laserstrahlung aus dem Laser S beaufschlagt wird; die Ablenkeinheit 10, hier als Scanner realisiert; ein das Teleskop 6 verwirklichendes Objektiv zur Fokussierung in das zu bearbeitende Gewebe, und die Steuereinheit 17.

Der Laser S erzeugt Laserpulse mit einer Dauer im fs-Bereich. Die Laserpulse gelangen zunächst in den Laserpulsmodulator 15, der (auf noch zu beschreibende Art) die Laserpulse gemäß einem Steuersignal der Steuereinheit 17 beeinflusst. Anschließend gelangen zumindest die Behandlungs-Laserpulse zum Scanner 10 und durch das Objektiv 6 in das Patientenauge 1. Sie werden dort fokussiert und erzeugen im Fokus 7 optische Durchbrüche. Der Modulator stellt die Energie der Laserpulse, d. h. die Fluence der einzelnen Laserpulse ein. Als Modulator kann ein AOM oder auch ein elektrooptischer Modulator (EOM), eine Pockelszelle, ein Flüssigkristallelement (LC-Element), ein faseroptisches Schaltelement oder ein variables Abschwächelement, beispielsweise Graufilter, verwendet werden.

Das laserchirurgische Instrument 1 kann nun in verschiedenen Betriebsarten arbeiten, die jeweils für sich eigenständig oder in Kombination realisiert sein können und die die Energie bzw. Fluence F jedes Laserpulses bzw. den örtlichen Abstand, mit dem die Laserpulse zur Erzeugung der Schnittfläche 9 aneinandergereiht werden, betreffen. Insbesondere können Kombinationen aus Energie (Pulsenergie) bzw. Fluence (Pulsfluence) und der örtliche Abstand, mit dem Impulse aneinander gereiht werden, gezielt gesteuert und so verschiedene Betriebsarten realisiert werden.

Es ist optional vorteilhaft eine oder mehrere solcher genannter Kombinationen als feste Parametersätze in einer Datenbank, auf die das Instrument Zugriff hat, zu speichern und wieder abrufbar zu machen. Dadurch kann vor oder während einer Materialbearbeitung der jeweilige Parametersatz abgerufen und zur beschriebenen Steuerung verwendet werden. Ein Parametersatz muss nicht zwingend einem bestimmten Materialbearbeitungsprozess zugeordnet sein. Vielmehr ist es unter anderem vorteilhaft, die Betriebsart in Abhängigkeit der gewünschten Bearbeitungszeit und/oder des Bearbeitungsortes und/oder der Bearbeitungsqualität zu wechseln. Im bearbeiteten Material liegen dann z.B. Bereiche vor, die gemäß einem für sie vorgesehenen Parametersatz bearbeitet werden.

Die Auswahl von Parametersätzen kann dabei manuell durch den Benutzer oder automatisch erfolgen. Es ist ebenfalls möglich, den Benutzers die Parametersätze nicht direkt auswählen zu lassen, sondern aus verschiedenen Eingaben des Benutzers den oder die günstigsten Parametersätze abzuleiten und während der Bearbeitung automatisch einzustellen. Eine andere Variante besteht darin, den gespeicherten Parametersatz abzurufen und gegebenenfalls in Abhängigkeit von Eingabewerten des Benutzers, und/oder anderen Messwerten zu modifizieren, bevor er angewendet wird.

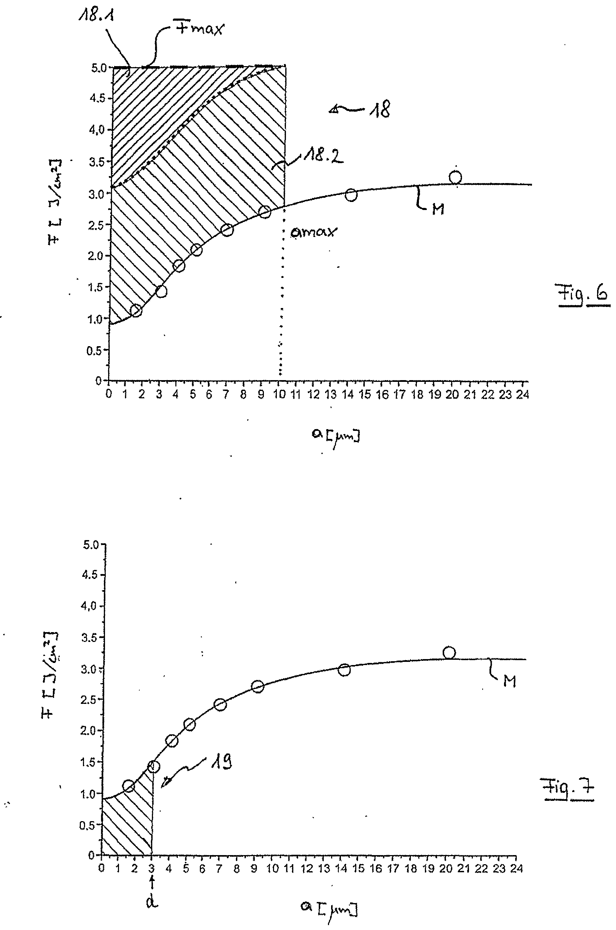

In Fig. 6 ist ein Schwellwert M als Kurve aufgetragen, der als Zusammenhang zwischen einem Abstand a, in dem die Wechselwirkungszentren der einzelnen Laserpulse in der Augenhornhaut 5 aneinandergereiht werden, und der Fluence F jedes Laserpulses wiedergibt. Nur bei einer Fluence über dem Schwellwert entsteht ein optischer Durchbruch mit anschließender Plasmablase.

Die an der Kurve gezeichneten kreisförmigen Eintragungen stammen aus experimentellen Vermessungen und stellen Messpunkte dar. Die Vermessungen erfolgten mit einer Pulslänge von 300 fs und einem Spotdurchmesser des Fokus 7 von 3 µm.

Das Instrument 1 kann in einem Betriebsgebiet 18 gemäß Fig. 6 betrieben werden, das durch verschiedene Randbedingungen definiert werden kann. Die unterschiedlichen Definitionen entsprechen unterschiedlichen Varianten der Erfindung. Alle Varianten nutzen den Verlauf des Schwellwertes M für die Fluence F als Funktion des Abstandes a aus. Diese Abhängigkeit ist näherungsweise durch folgende Formel gegeben: M = 3,3 J/cm² - (2,4 J/cm²) / (1 + (a/r²)²), wobei r ein Parameter ist, der die mittlere Reichweite der Beeinflussung wiedergibt und zwischen 3 und 10 µm, vorzugsweise 5 µm liegt.

In einer ersten Variante arbeitet das Instrument 1 mit einem Abstand a der Laserfoki 7, d. h. der Wechselwirkungszentren, der unter einem Maximalwert amax = 10µm liegt. Ab diesem Wert fällt die Kurve für den Schwellwert M zu niedrigeren Abständen a hin deutlich ab, so dass es möglich ist, mit einer deutlich reduzierten Fluence F zu arbeiten.

In einer zweiten Variante wird mit einer Obergrenze Fmax für die Fluence F gearbeitet. Der Wert hierfür ist 5 J/cm².

In einer Kombination der ersten und der zweiten Variante gilt sowohl a ≤ amax als auch F ≤ Fmax. Die Abstände der Wechselwirkungszentren sowie die Fluence der Laserpulse befinden sich also innerhalb des aus noch zu erläuternden Teilflächen 18.1 und 18.2 aufgebauten Gebietes. Da das laserchirurgische Instrument 1 in beiden Varianten für sich wie auch in der Kombination dieser beiden Varianten jeweils optische Durchbrüche im Material, beispielsweise der Hornhaut 5, erzeugt, liegt die Fluence F natürlich immer über dem Schwellwert M, da erst oberhalb dieses Schwellwertes jeder Laserpuls gesichert einen optischen Durchbruch 8 erzeugt.

Eine dritte Variante wandelt die zweite Variante nun dahingehend ab, dass die Fluence F jedes Laserpulses den Schwellwert M nur maximal um eine Überschussenergie überschreitet, die zwischen 3 und 3,5 J/cm² liegt. Die Fluence F wird dann unter der gepunkteten Linie der Fig. 6 gehalten, die die Bereiche 18.1 und 18.2 voneinander trennt. Natürlich kann auch die dritte Variante mit der ersten Variante kombiniert werden, wodurch Fluence F und Abstand a im schraffierten Gebiet 18.2 liegen.

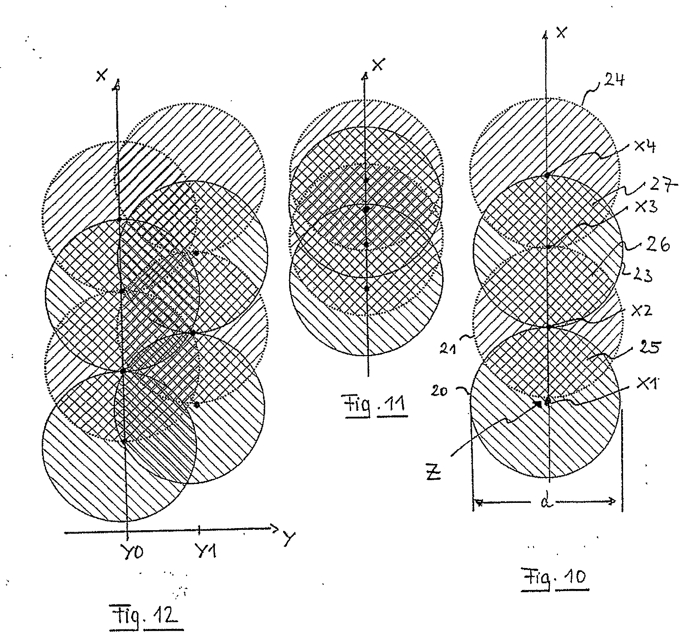

In einer anderen Ausgestaltung arbeitet das laserchirurgische Instrument 1 mit Laserpulsen, die nicht jeder gesichert einen optischen Durchbruch 8 erzeugen. Um dennoch eine Materialtrennung zu erreichen, sind die Wechselwirkungszentren in einem Abstand a aneinander angereiht, der geringer ist, als der Durchmesser d des Laserfokus, also geringer als die Größe der Wechselwirkungszonen. Diese Arbeitsweise ist in den Fig. 10- 12 näher gezeigt.

Fig. 10 zeigt in einem eindimensionalen Beispiel die Anordnung der Wechselwirkungszentren Z, die der Lage des (theoretischen) Fokuspunkts entsprechen. Jede Wechselwirkung wird durch einen Laserpuls erzeugt, wobei der Fokus 7 z. B. beugungsbegrenzt ist und den Durchmesser d hat, beispielsweise 3 µm, wie es in Fig. 7 angenommen ist. Die Wechselwirkungszentren, d. h. das Zentrum der fokussierten Laserstrahlung wird nun so verschoben, dass benachbart abgedeckte Wechselwirkungszonen 20, 21, 23 und 24 jeweils mit ihren unmittelbaren Nachbarn überlappen. Es gibt somit Überdeckungsbereiche 25, 26, 27, die jeweils von zwei Wechselwirkungszonen abgedeckt sind. Die in eine Wechselwirkungszone eingebrachte Energie liegt unter dem Schwellwert M, so dass jede der Wechselwirkungszonen 20 - 24 für sich nicht gesichert einen optischen Durchbruch bewirkt. Aufgrund der Überdeckung wird aber dennoch eine materialtrennende Wirkung erreicht. Wesentlich für diese Arbeitsweise ist also, dass der Abstand zwischen den Koordinaten der Wechselwirkungszentren geringer ist, als die Ausdehnung d der Wechselwirkungszonen. In Fig. 10 ist deutlich zu sehen, dass der Abstand zwischen den einzelnen Koordinaten X1, X2, X3 und X4 etwa dem halben Durchmesser d der Wechselwirkungszonen 20 - 24 entspricht, wodurch eine einfache Überdeckung gegeben ist.

Fig. 11 zeigt eine engere Staffelung der Wechselwirkungszonen, so dass im Endeffekt eine vierfache Überdeckung der Wechselwirkungszonen gegeben ist. Dies erlaubt eine weitere Absenkung der Fluence F.

Fig. 12 veranschaulicht, dass die Darstellung der Figuren 10 und 11 lediglich der Einfachheit halber nur eindimensional, d. h. nur unter Berücksichtigung der x-Koordinate dargestellt sind. Eine Verschiebung der sich gegenseitig in x-Richtung überlagernden Wechselwirkungszonen in y-Richtung erreicht weitere Überdeckungen, so dass trotz der an und für sich nur einfachen Überdeckung in x-Richtung je nach Beabstandung in y-Richtung eine drei- oder fünffache Überdeckung von Wechselwirkungszonen erreicht wird. Die Wahl der Abstände in x-Richtung bzw. y-Richtung erlaubt hier beliebige Überdeckungsfaktoren (2, 3, 4, 5, 6, 7, ...).

Im Ergebnis arbeitet das Instrument 1 im Betriebsbereich 19, der dadurch gekennzeichnet ist, dass der Abstand zwischen zwei aufeinanderfolgenden Wechselwirkungszentren geringer ist, als die Ausdehnung der Wechselwirkungszonen bzw. die Fokusspotgröße und dass die Fluence F unter dem zur Erzeugung optischer Durchbrüche erforderlichen Schwellwert M liegt.

Praktisch hat sich ein Abstand der Laserfoki bzw. Wechselwirkungszentren von ca. 3 - 5 µm als gut geeignet erwiesen, um mit möglichst geringer Pulsenergie und begrenztem Zeitaufwand Schnitte hoher Qualität zu erzeugen.

Bei einem laserchirurgischen Instrument 1, das sehr feine Schnitte erzeugt, beispielsweise wenn die eben geschilderten niedrigen Fluence-Werte für die Laserpulse verwendet werden, ist der Schnitt auch direkt nach der Erzeugung nicht sichtbar, entweder, weil Plasma- bzw. Gasblasen auftreten, die kleiner und kurzlebiger sind, als beim Betrieb außerhalb des Bereichs 18, oder weil überhaupt keine Blasen entstehen (bei Betrieb im Bereich 19). Die Präparation des isolierten Schnittes, beispielsweise mittels eines Spatels kann dann erschwert sein. Eine für viele Anwendungen verwendete manuelle Prozedur, bei der mit einem Spatel bzw. anderen Werkzeugen Restbrücken, die in der Schnittfläche noch nicht vollständig getrennt wurden, durchstoßen werden, kann bei einem glatten Schnitt sehr schwierig werden.

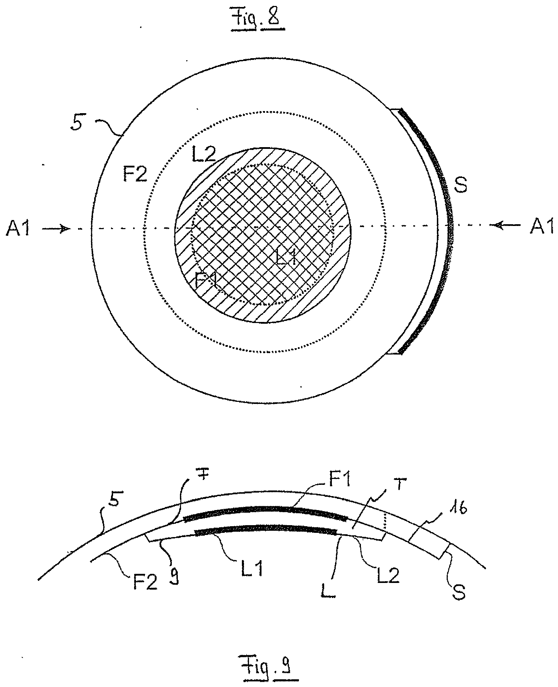

Um dies zu vermeiden, führt das Steuergerät 17 des laserchirurgischen Instrumentes 1 beispielsweise die in den Fig. 8 und 9 dargestellte Schnittzerlegung aus. Die Schnittfläche wird in Teilschnittflächen unterschiedlicher Feinheit zerlegt. Diese Teilschnittflächen werden unterschiedlich glatt geschnitten, so dass Bereiche bestehen, in denen die Schnittfläche optisch besser sichtbar ist, als in anderen.

Fig. 8 zeigt eine Draufsicht auf die Augenhornhaut 5 des Patientenauges 1 und Fig. 9 eine Schnittdarstellung entlang der Linie A1-A1 der Fig. 8. Wie zu sehen ist, ist die Schnittfläche 9 so gelegt, dass sie das Teilvolumen T isoliert, wie schon in Fig. 3 schematisch angedeutet war. Die Schnittfläche 9 besteht nun aus einem anterioren Abschnitt F und einem posterioren Abschnitt L. Der anteriore Abschnitt F wird über einen seitlich öffnenden Schnitt 16 zu einer Randöffnung S geführt, der zur Hornhautoberfläche führt. Das linsenförmige Teilvolumen T liegt also nach Ausführung der Schnittfläche 9 mit den Abschnitten F, L, 16 und S in einer durch die Randöffnung S gebildeten Tasche.

Damit ein Operateur diese Tasche mit einem Spatel oder einem anderen chirurgischen Instrument abfühlen kann, um eventuelle Gewebsbrücken zwischen dem linsenförmigen Teilvolumen T und dem Rest der Hornhaut 5 zu durchtrennen, ist der anteriore Abschnitt F wie auch der posteriore Abschnitt L jeweils in zwei Teilbereiche zerlegt. Ein Kernbereich F1 bzw. L1, der im Wesentlichen kreisförmig ist, wird jeweils von einem ringförmigen Randbereich F2 bzw. L2 umgeben. Im Kernbereich, der nahe der optischen Sehachse liegt, wird mit geringer Plasmablasengröße, d. h. mit einer feinen Schnittführung gearbeitet. Dies kann beispielsweise durch Betrieb in den Bereichen 18 bzw. 19 der Fig. 6 und 7 erfolgen. In den (ringförmigen) Randbereichen L2 und F2 wird dagegen ein vergleichsweise gröberer Schnitt erzeugt, beispielsweise indem bewusst außerhalb der Bereiche 18 oder 19 gearbeitet wird, so dass relativ große Plasmablasen entstehen. Die Schnittfläche ist somit in diesen Randbereichen sehr viel rauher und für den Operateur einfach zu erkennen.

Vorzugsweise sind die Durchmesser der zentralen Bereiche F1 und L1 größer als der Pupillendurchmesser P des behandelten Auges. Dadurch liegen die mit einer rauheren Schnittführung bearbeiteten Randbereiche F2 und L1 außerhalb des für die optische Wahrnehmung benutzten Bereiches der Hornhaut 5 und wirken sich nicht störend aus. Der Zweck der Zerlegung der Abschnitte L und F besteht darin, durch unterschiedliche Bearbeitung zugleich den Aspekt maximaler Schnittgenauigkeit als auch guter Handhabbarkeit durch Sichtbarkeit des Schnittes im Randbereich zu erreichen.

Arbeitet man zur Materialtrennung mit Plasmablasen, liegt die Energie der Laserpulse oberhalb des Schwellwertes M. Die Gestalt der Blasen, die das Ergebnis der Absorption der Laserenergie im Gewebe sind, unterliegt einer zeitlichen Änderung, wie bereits erwähnt. An eine erste Phase der Einzelblasenentstehung schließt sich eine Phase der Blasenagglomeration zusammen, in der sich mehrere Einzelblasen zu größeren Makroblasen zusammenschließen. Als letzte Phase ist schließlich die Dissipation zu verzeichnen, bei welcher der Gasgehalt der Makroblasen vom umliegenden Gewebe aufgenommen wird, bis die Blasen schließlich wieder vollends verschwunden sind. Makroblasen haben nun die störende Eigenschaft, das umliegende Gewebe zu deformieren. Wird in das deformierte Gewebe an eine bestimmte Position ein weiteres Wechselwirkungszentrum als Beginn einer Plasmablase gelegt, so verändert sich die Lage des Wechselwirkungszentrums und mithin die Lage der dadurch bewirkten Gewebetrennung, sobald die Dissipationsphase einsetzt, in der die Blasen verschwinden und sich das deformierte Gewebe (zumindest teilweise) relaxiert. Da sich die Makroblasen erst nach einer charakteristischen Zeit bilden und nicht schon direkt nach Einbringung der Laserpulsenergie vorhanden sind, ist für eine Variante des laserchirurgischen Instrumentes 1 vorgesehen, dass die Zeit zwischen der Applikation der Laserenergie in zwei sich potentiell beeinflussenden Gebieten des Gewebes so gering gehalten wird, dass sie kleiner ist, als eine charakteristische Zeitdauer, welche für die Bildung von Makroblasen erforderlich ist.

Bei der Isolierung des linsenförmigen Teilvolumens T sind einander störend beeinflussende Gebiete der posteriore und der anteriore Abschnitte der Schnittfläche 9 im Bereich der optischen Sehachse. Erzeugt man den Schnitt im anterioren Abschnitt F der Schnittfläche 9 erst zu einem Zeitpunkt, zu dem der zuvor bearbeitete posteriore Abschnitt L bereits Makroblasen aufweist, liegt die Schnittfläche des anterioren Abschnittes F in deformiertem Gewebe. Das Ergebnis wäre nach Relaxation des Gewebes eine unerwünschte Welligkeit der Schnittfläche 9 im anterioren Abschnitt F. Das laserchirurgische Instrument 1 erzeugt deshalb die Schnittfläche im anterioren Abschnitt F und im posterioren Abschnitt L in einem zeitlichen Abstand, der geringer ist, als die charakteristische Zeitdauer, in der Makroblasen entstehen. Diese Zeitdauer liegt typisch bei etwa 5 s.

Eine Möglichkeit dies zu bewerkstelligen liegt darin, den anterioren sowie den posterioren Abschnitt in entsprechende Teilflächen aufzuteilen und bei der Schnittflächenerzeugung zwischen den Teilflächen des posterioren bzw. anterioren Abschnittes zu wechseln, so dass zumindest im zentralen Bereich die charakteristische Zeitdauer bei der Erzeugung der Teilflächen, posterior und anterior, nicht überschritten wird. Eine andere Möglichkeit liegt in einer geeigneten Aneinanderreihung der Wechselwirkungszentren. So kann beispielsweise zuerst der posteriore Abschnitt L mit einer von außen nach innen zur optischen Sehachse führenden Spirale und direkt anschließend der anteriore Abschnitt F mit einer von der Sehachse nach außen laufenden Spirale geschnitten werden. Dann liegen zumindest in einem Kernbereich rund um die Sehachse die erzeugten Wechselwirkungen innerhalb des durch die charakteristische Zeitdauer vorgegebenen Zeitfensters, so dass keine Makroblasen-Beeinflussung bei der Bearbeitung des anterioren Abschnittes gegeben ist.

Bei der Aufteilung der Teilflächen, die das laserchirurgische Instrument 1 gesteuert durch die Steuereinrichtung 17 vornimmt, ist dafür Sorge getroffen, dass ein zu bearbeitender posteriorer Bereich nicht von einer als Streuzentrum wirkenden, bereits bearbeiteten anterioren Fläche oder Wechselwirkungszone gestört wird.

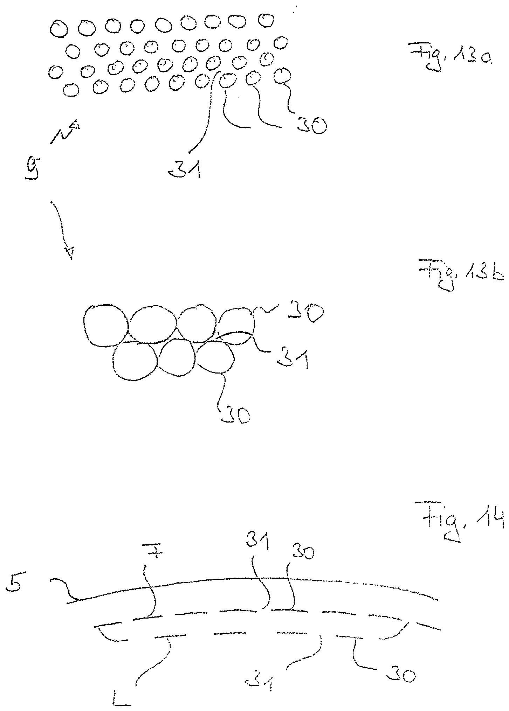

Die Schnittflächenerzeugung kann in einer Abwandlung und/oder Ergänzung der soeben beschriebenen Konzepte auch derart ausgebildet werden, dass keine vollständige Trennung der Material- bzw. Gewebsschichten erfolgt, sondern lediglich eine die Schnittflächengeometrie festlegende Perforation. Dies ist in Draufsicht schematisch in den Figuren 13a und 13b gezeigt. Die Schnittfläche 9 trennt hier nicht vollständig, sondern besteht aus Bereichen 30, in denen das Gewebe getrennt ist, und Materialbrücken 31, in denen noch keine Gewebstrennung erfolgte. Dazu kann jede geeignete Einbringung der Laserstrahlung verwendet werden, insbesondere ist es möglich, die Abstände, Energien, Überdeckungsgrade, etc. von Laserpulsen geeignet zu wählen. Die Art der Perforation kann dabei unterschiedlichst ausfallen. Es sind regelmäßige Muster von Materialbrücken 31 zwischen den getrennten Bereichen (Perforationen) 30 denkbar, wie auch unregelmäßig angeordnete Materialbrücken 31. Figur 13b zeigt eine exemplarische Schnittflächengestaltung, bei der die Bereiche 30 in Art einer dichtesten Kugelpackung aneinander liegen, in deren Zwischenräume die Materialbrücken 31 bestehen bleiben. Somit kann durch geeignete Einbringung der Laserpulse ein regelmäßiges oder unregelmäßiges Muster mit Materialbrücken 31 gebildet werden, wobei innerhalb der Schnittfläche abschnittsweise unterschiedliche Arten der Perforation, d.h. unterschiedliche Lage, Stärke und/oder Häufigkeit von Materialbrücken gewählt werden kann.

Figur 14 zeigt eine Schnittdarstellung ähnlich der Figur 9, wobei nun sowohl die posteriore Fläche L als auch die anteriore Fläche F durch Perforationen ausgebildet sind, also Bereiche 30, in denen das Gewebe getrennt ist, mit Materialbrücken 31 abwechseln. Figur 14 zeigt weiter exemplarisch, dass in der posterioren Fläche L die Materialbrücken stärker sind, als in der anterioren Fläche F. Klappt der Chirurg somit die durch die anteriore Fläche F gebildete Lamelle zur Entnahme des Lentikels ab, ist sichergestellt, dass das Lentikel L sich zuerst an der anterioren Fläche F vom Gewebe löst und nicht an der posterioren Fläche L. Dies ermöglicht es dem Chirurg, das Lentikel einfach zu entnehmen.

Natürlich kann die in den Figuren 13a, 13b und 14 gezeigte Perforation zur Schnittflächenausbildung auch mit herkömmlicher, vollständig trennender Schnittflächenausbildung kombiniert werden, wobei in der Augenchirurgie zu bevorzugen ist, dass innerhalb der üblichen Pupillengröße durchgängig eine Perforation in der Schnittflächengeometrie ausgeführt wird, da die optische Qualität dann besser ist.

Die geschilderten Schnittformen, Flächenaufteilungen, etc. werden vom laserchirurgischen Instrument unter Steuerung der Steuereinrichtung 17 vorgenommen. Die Steuereinrichtung 17 bewirkt den Betrieb des laserchirurgischen Instrumentes 1 auch mit den hier beschriebenen Verfahrensmerkmalen.

Soweit vorangehend Ausgestaltungen des laserchirurgischen Instrumentes geschildert sind, können diese allein wie auch in Kombination realisiert sein, je nach konkreter Realisierung des laserchirurgischen Instrumentes 1. Auch kann das Instrument 1 anstelle eines Einsatzes in der Laserchirurgie auch für die nicht chirurgische Materialbearbeitung verwendet, beispielsweise bei der Erzeugung von Wellenleitern oder der Bearbeitung flexibler Materialien.

## Patentansprüche

1. Vorrichtung zur Materialbearbeitung mittels Laserstrahlung, mit einer Laserstrahlungsquelle (S), die geeignet ist, gepulste Laserstrahlung (3) zur Wechselwirkung mit dem Material (5) abzugeben, einer die gepulste Bearbeitungs-Laserstrahlung (3) in das Material (5) auf ein Wechselwirkungszentrum (7) fokussierenden Optik (6), einer die Lage des Wechselwirkungszentrums im Material (5) verstellenden Scaneinrichtung (10), wobei jeder Bearbeitungs-Laserpuls in einer das ihm zugeordnete Wechselwirkungszentrum (7) umgebenden Zone (8) mit dem Material (5) wechselwirkt, so dass in den Wechselwirkungszonen (8) Material (5) getrennt wird, und einer Steuereinrichtung (17), welche die Scaneinrichtung (10) und die Laserstrahlungsquelle (S) so ansteuert, dass im Material (5) durch eine Vielzahl von Wechselwirkungszonen (8) eine Schnittfläche (9) entsteht, **dadurch gekennzeichnet, dass** die Steuereinrichtung (17) die Schnittfläche (9) umfassend einen anterioren (F) sowie einen posterioren Abschnitt (L) festlegt und die Scaneinrichtung (10) und die Laserstrahlungsquelle (S) so ansteuert, dass die Wechselwirkungszonen (8) im posterioren Abschnitt L entlang einer von außen nach innen führenden Spirale erzeugt werden.

2. Vorrichtung nach Anspruch 1, **dadurch gekennzeichnet, dass** die Wechselwirkungszonen (8) anschließend im anterioren Abschnitt F entlang einer nach außen laufenden Spirale erzeugt werden.

3. Verfahren zur nicht-chirurgischen Materialbearbeitung mittels Laserstrahlung, bei dem gepulste Laserstrahlung (3) erzeugt, zur Wechselwirkung ins Material (5) auf Wechselwirkungszentren (7) fokussiert wird, und die Lage der Wechselwirkungszentren (7) im Material (5) verstellt wird, wobei jeder Bearbeitungs-Laserpuls in einer das ihm zugeordnete Wechselwirkungszentrum (7) umgebenden Zone (8) mit dem Material (5) wechselwirkt, so dass in den Wechselwirkungszonen (8) Material (5) getrennt wird, und eine Schnittfläche (9) im Material (5) durch Aneinanderreihen von Wechselwirkungszonen (8) erzeugt wird, **dadurch gekennzeichnet,** die Steuereinrichtung (17) die Schnittfläche (9) eine anterioren (F) sowie einen posterioren Abschnitt (L) umfasst und die Wechselwirkungszonen (8) im posterioren Abschnitt L entlang einer von außen nach innen führenden Spirale erzeugt werden.

4. Verfahren nach Anspruch 3, **dadurch gekennzeichnet, dass** die Wechselwirkungszonen (8) anschließend im anterioren Abschnitt F entlang einer nach außen laufenden Spirale erzeugt werden.

5. Verfahren zum Bereitstellen von Datensätzen, wobei die Datensätze unterschiedliche Betriebsparameter für einzelne Bereiche zu bearbeitenden Materials vorgeben für eine Vorrichtung zur Materialbearbeitung, die aufweist
- eine Laserstrahlungsquelle (S), die gepulste Laserstrahlung (3) zur Wechselwirkung mit dem Material (5) abgibt,
- eine die gepulste Bearbeitungs-Laserstrahlung (3) in das Material (5) auf ein Wechselwirkungszentrum (7) fokussierenden Optik (6), eine die Lage des Wechselwirkungszentrums im Material (5) verstellenden Scaneinrichtung (10), wobei jeder Bearbeitungs-Laserpuls in einer das ihm zugeordnete Wechselwirkungszentrum (7) umgebenden Zone (8) mit dem Material (5) wechselwirkt, so dass in den Wechselwirkungszonen (8) Material (5) getrennt wird, und
- eine Steuereinrichtung (17), welche die Scaneinrichtung (10) und die Laserstrahlungsquelle (S) so ansteuert, dass im Material (5) durch eine Vielzahl von Wechselwirkungszonen (8) eine Schnittfläche (9) entsteht,
**dadurch gekennzeichnet, dass**
die Datensätze für die Steuereinrichtung (17) die Schnittfläche (9) umfassend einen anterioren (F) sowie einen posterioren Abschnitt (L) festlegen und die Steuereinrichtung (17) zur Ansteuerung der Scaneinrichtung (10) und der Laserstrahlungsquelle (S) so steuern, dass die Wechselwirkungszonen (8) im posterioren Abschnitt L entlang einer von außen nach innen führenden Spirale erzeugt werden.

6. Verfahren nach Anspruch 5, **dadurch gekennzeichnet, dass** die Datensätze die Wechselwirkungszonen (8) anschließend im anterioren Abschnitt F entlang einer nach außen laufenden Spirale vorgeben.

## Claims

1. A device for material processing by means of laser radiation, said device comprising a source of laser radiation (S) adapted to emit pulsed laser radiation (3) for interaction with the material (5); optics (6) focusing the pulsed processing laser radiation (3) to a center of interaction (7) in the material (5); a scanning unit (10) shifting the position of the center of interaction within the material (5), wherein each processing laser pulse interacts with the material (5) in a zone (8) surrounding the center of interaction (7) assigned to said laser pulse so that material (5) is separated in the zones of interaction (8); and a control unit (17) which controls the scanning unit (10) and the source of laser radiation (S) such that a cut surface (9) is produced in the material (5) by a plurality of zones of interaction (8), **characterized in that** the control unit (17) defines the cut surface (8) to comprise an anterior (F) and a posterior section (L) and controls the scanning unit (10) and the source of laser radiation (S) such that the zones of interaction (8) are generated in the posterior section L along a spiral leading from the periphery to the center.

2. The device of claim 1, **characterized in that** subsequently the zones of interaction (8) are generated in the anterior section F along a spiral leading outwards.

3. A method of non-surgical material processing by means of laser radiation, wherein pulsed laser radiation (3) is generated, is focused for interaction into the material (5) and on centers of interaction (7), and the position of the centers of interaction (7) is shifted in the material (5), each processing laser pulse interacting with the material (5) in a zone (8) surrounding the center of interaction (7) assigned to said laser pulse, so that the material (5) is separated in the zones of interaction (8) and a cut surface (9) is formed in the material (5) by a plurality of zones of interaction (8), **characterized in that** the cut surface (9) comprises an anterior (F) and a posterior section (L) and that the zones of interaction (8) are generated in the posterior section L along a spiral leading from the periphery to the center.

4. The method according to claim 3, **characterized in that** subsequently the zones of interaction (8) are generated in the anterior section F along a spiral leading outwards.

5. A method for providing data sets, which data sets provide different operation parameters for individual sections of material to be processed by a device for material processing using laser radiation, said device comprising
- a source of laser radiation (S) adapted to emit pulsed laser radiation (3) for interaction with the material (5);
- optics (6) focusing the pulsed processing laser radiation (3) to a center of interaction (7) in the material (5); a scanning unit (10) shifting the position of the center of interaction within the material (5), wherein each processing laser pulse interacts with the material (5) in a zone (8) surrounding the center of interaction (7) assigned to said laser pulse so that material (5) is separated in the zones of interaction (8); and
- a control unit (17) which controls the scanning unit (10) and the source of laser radiation (S) such that a cut surface (9) is produced in the material (5) by a plurality of zones of interaction (8),
**characterized in that**
the data sets define to the control unit (17) the cut surface (8) to comprise an anterior (F) and a posterior section (L) and control the control unit (17) for controlling the scanning unit (10) and the source of laser radiation (S) such that the zones of interaction (8) are generated in the posterior section L along a spiral leading from the periphery to the center.

6. The method according to claim 5, **characterized in that** the data sets subsequently provide the zones of interaction (8) in anterior section F along a spiral leading outwards.

## Revendications

1. Dispositif de traitement de matériau au moyen d'un rayonnement laser, comprenant une source de rayonnement laser (S) qui est conçue pour délivrer un rayonnement laser pulsé (3) destiné à interagir avec le matériau (5), une optique (6) qui concentre le rayonnement laser de traitement pulsé (3) dans le matériau (5) sur un centre d'interaction (7), un appareil de balayage (10) qui modifie la position du centre d'interaction dans le matériau (5), chaque impulsion laser de traitement interagissant avec le matériau (5) dans une zone (8) qui entoure le centre d'interaction (7) qui lui est associé, de sorte que du matériau (5) soit enlevé dans les zones d'interaction (8), et un appareil de commande (17) qui commande l'appareil de balayage (10) et la source de rayonnement laser (S) de telle sorte qu'une surface de coupe (9) est produite dans le matériau (5) par une pluralité de zones d'interaction (8), **caractérisé en ce que** l'appareil de commande (17) définit la surface de coupe (9) comportant une portion antérieure (F) ainsi qu'une portion postérieure (L) et commande l'appareil de balayage (10) et la source de rayonnement laser (S) de telle sorte que les zones d'interaction (8) dans la portion postérieure (L) sont générées le long d'une spirale qui mène de l'extérieur vers l'intérieur.

2. Dispositif selon la revendication 1, **caractérisé en ce que** les zones d'interaction (8) sont ensuite générées dans la portion antérieure (F) le long d'une spirale qui s'étend vers l'extérieur.

3. Procédé de traitement non chirurgical de matériau au moyen d'un rayonnement laser, selon lequel un rayonnement laser pulsé (3) est généré, concentré sur des centres d'interaction (7) en vue d'interagir avec le matériau (5), et la position des centres d'interaction (7) dans le matériau (5) est modifiée, chaque impulsion laser de traitement interagissant avec le matériau (5) dans une zone (8) qui entoure le centre d'interaction (7) qui lui est associé, de sorte que du matériau (5) soit enlevé dans les zones d'interaction (8), et une surface de coupe (9) étant produite dans le matériau (5) par l'alignement juxtaposé des zones d'interaction (8), caractérisé en ce l'appareil de commande (17) la surface de coupe (9) comporte une portion antérieure (F) ainsi qu'une portion postérieure (L) et les zones d'interaction (8) dans la portion postérieure (L) sont générées le long d'une spirale qui mène de l'extérieur vers l'intérieur.

4. Procédé selon la revendication 3, **caractérisé en ce que** les zones d'interaction (8) sont ensuite générées dans la portion antérieure (F) le long d'une spirale qui s'étend vers l'extérieur.

5. Procédé de fourniture de jeux de données, les jeux de données prédéfinissant différents paramètres de fonctionnement pour les zones individuelles d'un matériau à traiter pour un dispositif de traitement de matériau, lequel possède
- une source de rayonnement laser (S) qui délivre un rayonnement laser pulsé (3) destiné à interagir avec le matériau (5),
- une optique (6) qui concentre le rayonnement laser de traitement pulsé (3) dans le matériau (5) sur un centre d'interaction (7), un appareil de balayage (10) qui modifie la position du centre d'interaction dans le matériau (5), chaque impulsion laser de traitement interagissant avec le matériau (5) dans une zone (8) qui entoure le centre d'interaction (7) qui lui est associé, de sorte que du matériau (5) soit enlevé dans les zones d'interaction (8), et
- un appareil de commande (17) qui commande l'appareil de balayage (10) et la source de rayonnement laser (S) de telle sorte qu'une surface de coupe (9) est produite dans le matériau (5) par une pluralité de zones d'interaction (8),
**caractérisé en ce que**
les jeux de données pour l'appareil de commande (17) définissent la surface de coupe (9) comportant une portion antérieure (F) ainsi qu'une portion postérieure (L) et commandent l'appareil de commande (17) pour la commande de l'appareil de balayage (10) et de la source de rayonnement laser (S) de telle sorte que les zones d'interaction (8) dans la portion postérieure (L) sont générées le long d'une spirale qui mène de l'extérieur vers l'intérieur.

6. Procédé selon la revendication 5, **caractérisé en ce que** les jeux de données prédéfinissent ensuite les zones d'interaction (8) dans la portion antérieure (F) le long d'une spirale qui s'étend vers l'extérieur.
